**Europäisches Patentamt**

**European Patent Office**

**Office européen des brevets**

(19)

(11) Publication number: **0 323 424**
**A2**

# (12) EUROPEAN PATENT APPLICATION

(21) Application number: **88850434.7**

(22) Date of filing: **23.12.88**

(51) Int. Cl.⁴: **C 07 K 7/00**
**G 01 N 33/68, A 61 K 37/02**

(30) Priority: **30.12.87 SE 8705197**

(43) Date of publication of application:
**05.07.89 Bulletin 89/27**

(84) Designated Contracting States:
**AT BE CH DE ES FR GB GR IT LI LU NL SE**

(71) Applicant: **Blomberg, Jonas**
**Storgatan 5**
**S-23400 Lomma (SE)**

**Pipkorn, Rüdiger**
**Ugglumsvägen 12**
**S-43362 Partille (SE)**

**Klasse, Per Johan**
**Eldaregatan 2 A**
**S-22237 Lund (SE)**

(72) Inventor: **Blomberg, Jonas**
**Storgatan 5**
**S-23400 Lomma (SE)**

**Pipkorn, Rüdiger**
**Ugglumsvägen 12**
**S-43362 Partille (SE)**

**Klasse, Per Johan**
**Eldaregatan 2 A**
**S-22237 Lund (SE)**

(74) Representative: **Nilsson, Brita et al**
**AWAPATENT AB Box 5117**
**S-200 71 Malmö (SE)**

(54) Peptide corresponding to HIV-env 583-599, and analogs thereof and applications of the peptides.

(57) A peptide having the amino acid sequence

H-Leu-Gln-X-Arg-Ile-Leu-Ala-Val-Glu-Arg-Tyr-Leu-Lys-Asp-Y-Z-Leu-W

wherein
X represents a peptide bond or an amino acid residue selected from Ala, Thr, Val, Ser and Asn;
Y represents a peptide bond or an amino acid residue selected from Gln, Gly, Ala and Thr;
Z represents a peptide bond or an amino acid residue selected from Gln, Gly, Ala and Thr; and
W represents -OH or -NH₂;
is described. When X represents Ala, Y represents Gln and Z represents Gln, the peptide corresponds to a part of gp41, the transmembrane protein of HIV-1 (human immunodeficency virus type 1). Furthermore, there is disclosed the above defined peptides for diagnostic use in the determination of possible development in an HIV-infected patient of a disease caused by HIV, such as LAS, ARC or AIDS; the antibodies reacting with at least one of said peptides; a method of isolating antibodies reacting with at least one of said peptides; a method of determining possible development in an HIV-infected patient of a disease caused by HIV, wherein said peptides are used; a method of detecting immunosuppressive protein produced by HIV, wherein said peptides are used; a medicament for prophylactic or therapeutic use in the prevention or treatment of a disease caused by HIV, which comprises antibodies reacting with at least one of said peptides; and a vaccine composition comprising at least one of said peptides.

FIG.1

**Description**

## PEPTIDE CORRESPONDING TO HIV-env 583-599, AND ANALOGS THEREOF AND APPLICATIONS OF THE PEPTIDES

The present invention relates to a peptide corresponding to a part of gp41, the transmembrane protein of HIV-1 (human immunodeficency virus type 1), and analogs thereof. Furthermore, it relates to said peptide and said analogs for diagnostic use in the determination of possible development in an HIV-infected patient of a disease caused by HIV, such as LAS, ARC or AIDS; to the antibodies reacting with at least one of said peptide and analogs; to a method of isolating antibodies reacting with at least one of said peptide and analogs; to a method of determining possible development in an HIV-infected patient of a disease caused by HIV, wherein said peptide and analogs are used; to a method of detecting immunosuppressive protein produced by HIV, wherein said peptide and analogs are used; to a medicament for prophylactic or therapeutic use in the prevention or treatment of a disease caused by HIV, which comprises antibodies reacting with at least one of said peptide and analogs; and to a vaccine composition comprising at least one of said peptides and analogs.

### Background

Immunosuppression by HIV may be mediated both by a reduction in number of T helper lymphocytes and by impairment of their function. Much evidence supports killing and syncytia formation of lymphocytes, resulting from interactions between the HIV gp120 and the CD4-molecule, as a major mechanism behind the immune defects seen in AIDS patients (1, 2, 3,). Part of HIV gp41 has also been implicated in the cytotoxicity of HIV (4). However, T-helper cells from AIDS patients are not only low in number but qualitative defects have also been demonstrated (5, 6). B-lymphocyte dysfunction is also present in AIDS and has been attributed to both stimulatory and inhibitory direct effects of HIV (5-8). Soluble suppressor factors obtained from cultures of peripheral blood mononuclear cells from patients with AIDS have been shown to inhibit T-cell blastogenic responses to antigen and B-cell differentiation in vitro (9, 10).

Many mammalian retroviruses contain an evolutionarily conserved sequence in their transmembrane glycoproteins, e.g. p15E of FeLV and MuLV (11-17). A 17-amino acid peptide derived from this sequence in MuLV has revealed immunosuppressive properties and has 6 exact amino acid matches with the corresponding region of the transmembrane glycoprotein of HIV, gp41 (14).

We synthesized and purified a series of eight peptides derived from three regions of HIV gp41. The presence of antibodies to them was tested in an enzyme immunoassay. From the first region, a 17-amino acid peptide (HIV-env 583-599; pHIVIS) with the mentioned similarity to immunosuppressive sequences in other retroviruses was produced. As a comparison two peptides with four amino acid N-and C-terminal displacements relative to pHIVIS (HIV-env 579-595 and HIV-env 587-603) as well as its inverted sequence were also synthesized. A 21-amino acid peptide (HIV-env 586-606) from the same region and previously reported by others to detect antibodies in nearly all HIV-positive sera (18) was made as a further control.

From a second region we synthesized a 13 amino acid peptide (HIV-env 654-666) recently predicted to be an important epitope (19). From a third region (the carboxy terminus) two peptides (HIV-env 648-663 and HIV-env 654-663) were derived and produced. The latter region contains a sequence similarity with a putative receptor binding site of interleukin 2, and has been proposed to have an immunosuppressive activity (20).

A peptide corresponding to a part of gp41, the transmembrane protein of HIV-1 (human immunodeficiency virus type 1), inhibits stimulation of lymphocytes in vitro (37). The presence of antibodies reactive with this peptide, HIV-env 583-599, is now shown to be associated with health among HIV-seropositive persons. Furthermore, a single amino-acid change in this part of gp41 can confer resistence to neutralization by a human serum (45). We deleted single amino acids from HIV-env 583-599 and substituted pairs of threonines for the amino acids in the peptide HIV-env 581-599. These and four other peptides derived from the same region of gp41 were tested against 21 HIV-1-positive sera in an enzyme immunoassay.

To our knowledge, no one has previously reported a method using a peptide for determination of possible development in an HIV-infected patient of a disease caused by HIV.

### Description of the invention

In one aspect of the invention there is provided a peptide having the amino acid sequence

H-Leu-Gln-X-Arg-Ile-Leu-Ala-Val-Glu-Arg-Tyr-Leu-Lys--Asp-Y-Z-Leu-W

wherein
X represents a peptide bond or an amino acid residue selected from Ala, Thr, Val, Ser and Asn;
Y represents a peptide bond or an amino acid residue selected from Gln, Gly, Ala and Thr;
Z represents a peptide bond or an amino acid residue selected from Gln, Gly, Ala and Thr; and
W represents -OH or -NH$_2$.

When X represents Ala, Y represents Gln and Z represents Gln the peptide corresponds to a part of gp41, the transmembrane protein of HIV-1, and this peptide will in the specification be referred to as HIV-env 583-599 or as pHIVIS.

In another aspect of the invention there is provided a peptide for diagnostic use in the determination of possible development in an HIV infected patient of a disease caused by HIV, such as LAS, ARC or AIDS, and said peptide is chosen from the group consisting of peptides having the amino acid sequence

H-Leu-Gln-X-Arg-Ile-Leu-Ala-Val-Glu-Arg-Tyr-Leu-Lys-Asp-Y-Z-Leu-W

wherein
X represents a peptide bond or an amino acid residue selected from Ala, Thr, Val, Ser and Asn;
Y represents a peptide bond or an amino acid residue selected from Gln, Gly, Ala and Thr;
Z represents a peptide bond or an amino acid residue selected from Gln, Gly, Ala and Thr; and
W represents -OH or -NH$_2$.

In yet another aspect of the invention there is provided an antibody reacting with at least one peptide chosen from the group consisting of peptides having the amino acid sequence

H-Leu-Gln-X-Arg-Ile-Leu-Ala-Val-Glu-Arg-Tyr-Leu-Lys-Asp-Y-Z-Leu-W

wherein
X represents a peptide bond or an amino acid residue selected from Ala, Thr, Val, Ser and Asn;
Y represents a peptide bond or an amino acid residue selected from Gln, Gly, Ala and Thr;
Z represents a peptide bond or an amino acid residue selected from Gln, Gly, Ala and Thr; and
W represents -OH or -NH$_2$.

In an embodiment of this aspect of the invention said antibody is a monoclonal antibody. The antibodies according to the invention are optionally coupled to a carrier. Examples of suitable carriers are bovine serum albumin, plastic or glass surfaces or particles and gelatin particles.

In still another aspect of the invention there is provided a method of isolating antibodies reacting with at least one peptide chosen from the group consisting of peptides having the amino acid sequence

H-Leu-Gln-X-Arg-Ile-Leu-Ala-Val-Glu-Arg-Tyr-Leu-Lys-Asp-Y-Z-Leu-W

wherein
X represents a peptide bond or an amino acid residue selected from Ala, Thr, Val, Ser and Asn;
Y represents a peptide bond or an amino acid residue selected from Gln, Gly, Ala and Thr;
Z represents a peptide bond or an amino acid residue selected from Gln, Gly, Ala and Thr; and
W represents -OH or -NH$_2$;
from blood samples of HIV infected patients, wherein said blood samples are subjected to affinity chromatography; in a per se known manner.

The blood samples can be samples of whole blood or plasma.

In yet another aspect of the invention there is provided a method of determining possible development in an HIV infected patient of a disease caused by HIV, such as LAS, ARC or AIDS, with the aid of a sample of body fluid from said patient, wherein said sample is subjected to an immunoassay using as diagnostic antigen at least one peptide chosen from peptides having the amino acid sequence

H-Leu-Gln-X-Arg-Ile-Leu-Ala-Val-Glu-Arg-Tyr-Leu-Lys-Asp-Y-Z-Leu-W

wherein
X represents a peptide bond or an amino acid residue selected from Ala, Thr, Val, Ser and Asn;
Y represents a peptide bond or an amino acid residue selected from Gln, Gly, Ala and Thr;
Z represents a peptide bond or an amino acid residue selected from Gln, Gly, Ala and Thr; and
W represents -OH or -NH$_2$;
and in case said sample of body fluid contains antibodies which bind to said diagnostic antigen, the patient is not likely to have a disease caused by HIV or develop one within a few years' time.

The sample of body fluid can be of any body fluid having detectable amounts of antibodies which bind to the above defined diagnostic antigen. Preferably samples of whole blood or plasma are used. The expression "a few years' time" is used to indicate a period of about 2-3 years. The length of this period is not established.

In a preferred embodiment of this aspect of the invention the immunoassay is enzyme linked immunosorbent assay (ELISA).

In an additional aspect of the invention there is provided a method of detecting immunosuppressive protein produced by HIV in a sample of body fluid from an HIV infected patient, wherein said sample of body fluid is subjected to a competitive inhibition test using as a test peptide a radioactively, enzymatically or otherwise labelled peptide chosen from the group of peptides having the amino acid sequence

H-Leu-Gln-X-Arg-Ile-Leu-Ala-Val-Glu-Arg-Tyr-Leu-Lys-Asp-Y-Z-Leu-W

wherein
X represents a peptide bond or an amino acid residue selected from Ala, Thr, Val, Ser and Asn;
Y represents a peptide bond or an amino acid residue selected from Gln, Gly, Ala and Thr;

Z represents a peptide bond or an amino acid residue selected from Gln, Gly, Ala and Thr; and
W represents -OH or -NH$_2$;
and in case the binding of said test peptide to antibodies directed against at least one peptide of the above group of peptides is inhibited, said sample of body fluid contains immunosuppressive protein produced by HIV.

In yet another aspect of the invention there is provided a medicament for prophylactic or therapeutic use in the prevention or treatment of a disease caused by HIV, in which the active principle of said medicament comprises antibodies reacting with at least one peptide chosen from the group consisting of peptides having the amino acid sequence

H-Leu-Gln-X-Arg-Ile-Leu-Ala-Val-Glu-Arg-Tyr-Leu-Lys-Asp-Y-Z-Leu-W

wherein
X represents a peptide bond or an amino acid residue selected from Ala, Thr, Val, Ser and Asn;
Y represents a peptide bond or an amino acid residue selected from Gln, Gly, Ala and Thr;
Z represents a peptide bond or an amino acid residue selected from Gln, Gly, Ala and Thr; and
W represents -OH or -NH$_2$.

In one embodiment of this aspect of the invention the antibodies are polyclonal antibodies derived from blood samples of HIV infected human patients or apes, and in another embodiment said antibodies are monoclonal antibodies.

In a further aspect of the invention there is provided a vaccine composition, which comprises as an immunizing component at least one peptide chosen from the group consisting of peptides having the amino acid sequence

H-Leu-Gln-X-Arg-Ile-Leu-Ala-Val-Glu-Arg-Tyr-Leu-Lys-Asp-Y-Z-Leu-W

wherein
X represents a peptide bond or an amino acid residue selected from Ala, Thr, Val, Ser and Asn;
Y represents a peptide bond or an amino acid residue selected from Gln, Gly, Ala and Thr;
Z represents a peptide bond or an amino acid residue selected from Gln, Gly, Ala and Thr; and
W represents -OH or -NH$_2$; together with a nontoxic pharmaceutically acceptable carrier and/or diluent.

In an embodiment of this aspect of the invention the immunizing component is present in amount effective to protect a subject from a disease caused by HIV. In another embodiment of this aspect of the invention an adjuvant is included in an amount, which together with an amount of said immunizing component, is effective to protect a subject from a disease caused by HIV.

## Short description of the drawings

Figure 1 is a graph showing the frequency of IgG antibody reactivity with eight different peptides derived from the HIV gp41 sequence.

Figure 2 comprises three graphs showing the distribution of antibody reactivity in healthy and diseased HIV seropositive persons with the same peptides as in Figure 1 in relation to their sequence position and hydrophilicity.

Figure 3 comprises two graphs showing the quantitative distribution of reactivities to the same eight peptides as in Figure 1.

Figure 4 is a graph showing the EIA reactivities for 21 HIV-positive sera with five peptides derived from the nucleotide sequence of the HIV1 env gene.

Figure 5 is a graph showing the EIA reactivities with threonine-substituted peptides for 17 sera positive for HIV-env 581-599.

Figure 6 is a graph showing the EIA reactivities with deletion peptides corresponding to the peptides of Figure 5 for 17 sera positive for HIV-env 583-599.

## Description of experiments

The data obtained in our work are discussed in relation to the conjecture that antibodies to a possibly immunsuppressive portion of gp41 (pHIVIS) protect against progession to symptomatic forms of HIV infection, including AIDS.

## MATERIALS AND METHODS

### Peptide synthesis

Peptides were derived from the amino acid sequence of the HIV isolate HTLVIIIB (21). The synthesis followed the stepwise solidphase strategy developed by Merrifield (22) and were performed on an automated peptide synthesizer (430A, Applied Biosystems, Foster City, California, USA). For temporary amino terminal protection we used the base labile N$^a$-9-fluorenvlmethoxycarbonyl (FMOC) group. The side chains were blocked in the following ways: tert.-butyl for Asp, Glu, Ser, Thr and Tyr; 4-methoxy-2,3,6-trimethyl

4

benzenesulfonyl for Arg; tert.-butyloxycarbonyl for Lys; and trityl for His and Cys. A p-alkoxy-benzyl alcohol resin (23) was utilized throughout the synthesis. The synthetic peptides were cleaved from the resin with trifluoroacetic acid/thioanisol for 4 hours and purified to homogeneity by preparative HPLC (Waters, Bedford, Massachusetts, USA) using a reverse phase Nucleosil C18 250x10 mm column with different gradients (depending on peptide) of 10-90% solution B (acetonitrile/water 60/40) mixed with solution A (0.1% trifluoroacetic acid in water) at a flow rate of 6 ml/min. The amino acid proportions were ascertained by the Moore and Stein procedure. The purity, investigated by means of HPLC, was estimated in all cases to be at least 98%.

## Sera

We used sera from 46 persons confirmed to be HIV antibody positive by EIB. 32 of these subjects were healthy, 4 had acquired immunodeficiency syndrome (AIDS), 2 had AIDS-related complex (ARC) and 8 had lymphadenopaty syndrome (LAS) according to the CDC criteria (24). The peptides were tested against 17 HIV-antibody negative sera, whereof 2 were from patients with leukemia and the rest from healthy blood donors. Two peptides were tested against a further 72 HIV-negative sera, 6 from leukemia patients and 66 from healthy blood donors.

## Enzyme immunoassay

Enzyme immunoassay was carried out with modification of a previously described method (25). 100 µl per well of a solution of 10 µg peptide per ml PBS/1 mM sodium merthiolate (r) (pH 7.4) (PBS-M) or only PBS-M as a control were left to adsorb over night at 4°C in 96-well microtitration plates (Nunc Immunoplate II, Nunc, Roskilde, Denmark). 200 µl per well of a blocking solution containing 4% (wt/vol) bovine serum albumin (fraction V, Boehringer Mannheim, Mannheim, West Germany), 0.2% (wt/vol) gelatin (Difco Laboratories, Detroit, Michigan) in PBS-M (pH 7.4) was added, left for 6 h at ambient temperature and subsequently over night at 4°C. Before use the plates were washed four times with PBS/0.05% (wt/vol) Tween 20 (PBS-Tween)/0.1% (wt/vol) sodium azide. Sera were diluted in 3% (wt/vol) bovine serum albumin/0.2% (wt/vol) gelatin/0.05% (vol/vol) Tween 20 in PBS. 100 µl of serum diluted 1/20 were added to all wells and left to incubate for 1 h at ambient temperature on a shaker. The plates were washed 5 times with PBS-tween. 100 µl of peroxidase conjugated rabbit immunoglobulins to human IgG-gamma chains (Dakopatt, Denmark) diluted 1/400 in PBS-tween/0.2% gelatin (wt/vol) was added and incubated on a shaker for 1 h at room temperature. After washing 5 times with PBS-tween (100 µl of substrate solution per well (40 mg o-phenylenediamine/20 µl 30% $H_2O_2$ per 100 ml buffer (34.7 mM citric acid/66.7 mM $Na_2HPO_4$ solution, pH 5.0) were added. Following 1 h a of enzyme reaction at room temperature in darkness the plates were read in a Titertek Multiskan (Flow Laboratories, Irvine, Scotland) colorimeter at 450 nm.

## Densitometry and EIA titrations

Densitometry of enzyme immunoblot strips was performed on a random sample of the sera, as previously described (26) on a Bio-rad model 620 video densitometer (Bio-Rad, Richmond, California). Absorbance values were expressed in "color yield units" (26). A commercial enzyme immunoassay for detection of anti-HIV antibodies (Abbott anti-HTLVIII EIA, Abbott, North Chicago, Illinois) was used according to the instructions of the manufacturer, except for the serum dilution which was varied from $4x10^{-2}$ to $4x10^{-6}$.

## Statistical methods and computer programs

We used Wilcoxon's rank sum test and Fisher's exact test as included in the Microstat statistical package (Ecosoft, Indianapolis, USA). The PC/Gene program (Genofit, Geneva, Switzerland) was used for computation and plotting of Hopp-Woods' index (27), presented as a moving average over 6 amino acids.

## RESULTS

## Frequency of reactions

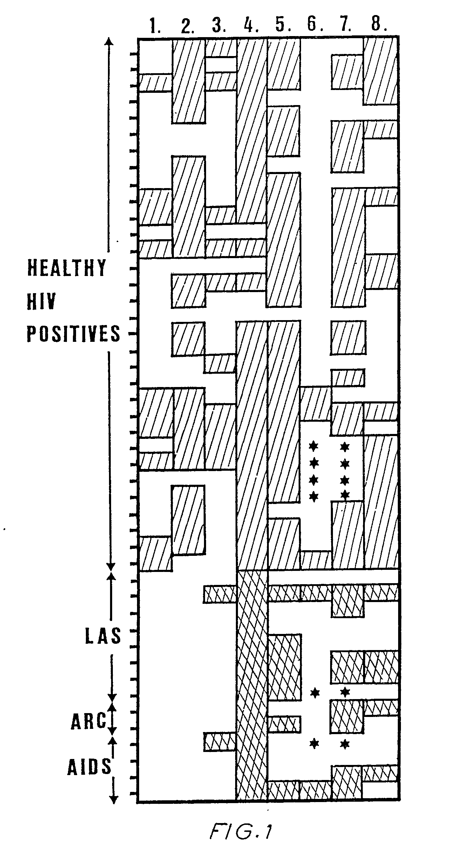

For each serum an absorbance value was calculated from the absorbances of three microtiter wells, as the difference between the average absorbance of two peptide coated wells and a well mock-coated with PBS-M. There was no difference in HIV specificity for the various peptides with an arbitrary cutoff value of 0.100 absorbance units, i.e. no HIV-negative serum exceeded this value for any peptide. A mosaic pattern of reactivity was however found with the HIV antibody positive sera (Fig 1). The most sensitive peptide for detection of HIV-seropositivty (as definied by EIB-positivity) was HIV-env 586-606, the longest peptide in the study: 43 out of 46 EIB-seropositive sera were positive for this peptide. The second most frequently reactive peptide was HIV-env 587-603: 36/46 reacted. 30/40 HIV-positive sera reacted with HIV-env 848-863 and 22/46 with HIV-env 854-863. 24/46 were positive for HIV-env 583-599 (pHIVIS) and 12/46 for its inverted counterpart, "HIVenv 599-583". The most N-terminally located peptide, HIV-env 579-595 gave 10 out of 46 reactions above cutoff. Only 5/40 sera reacted with HIV-env 654-666. We gradually increased the peptide concentration to 50 µg/ml without seeing any shifts in the relative absorbances obtained with the eight peptides (data not shown).

The specificities of HIV-env 583-599 (pHIVIS) and its inversion were further tested against 66 HIV-negative blood donor sera and 6 HIV-negative sera from patients with leukemia. All yielded absorbances below the stipulated cutoff (data not shown).

Statistical analysis and comparison of groups

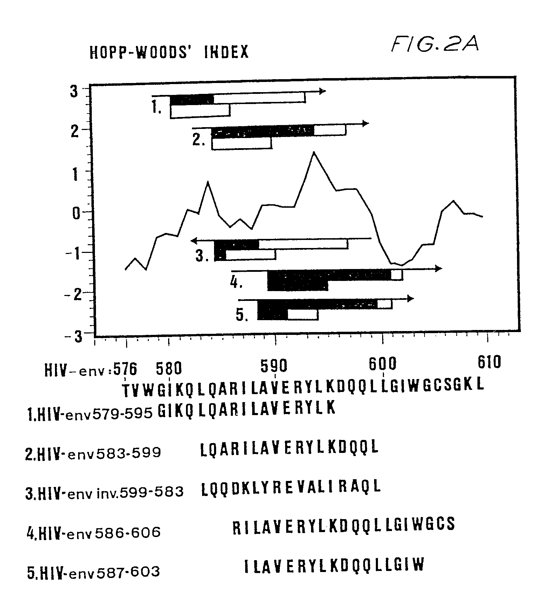

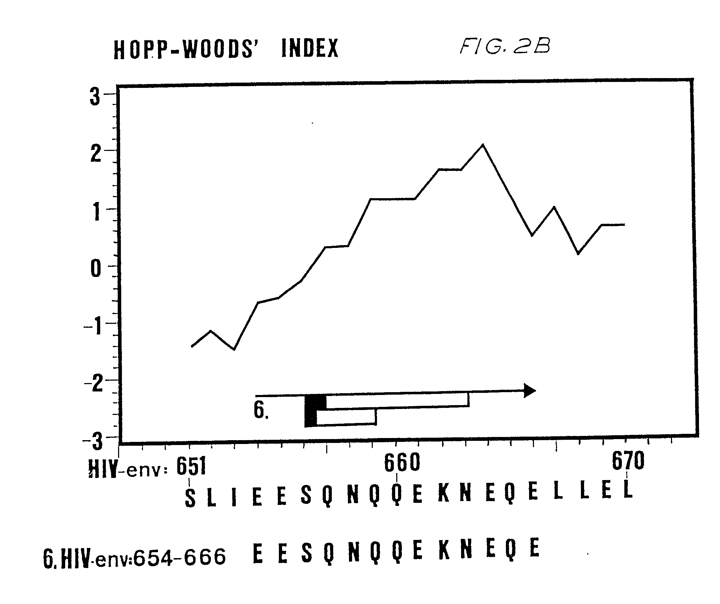

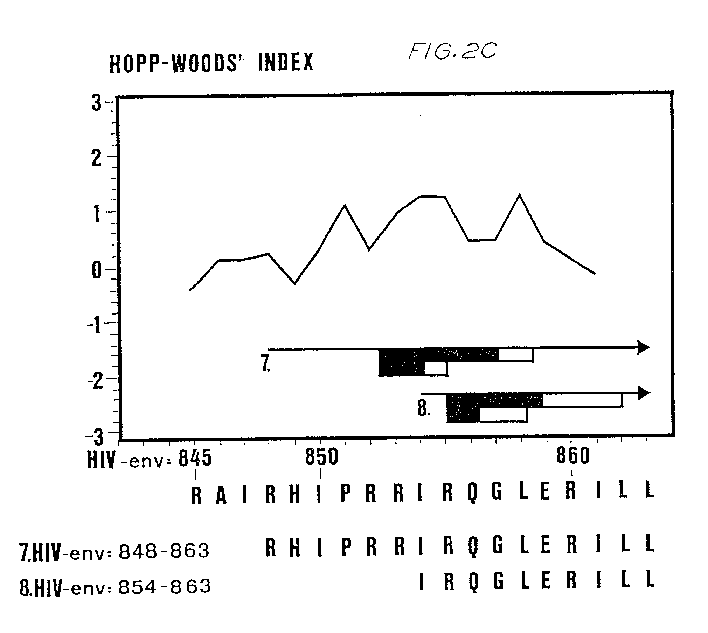

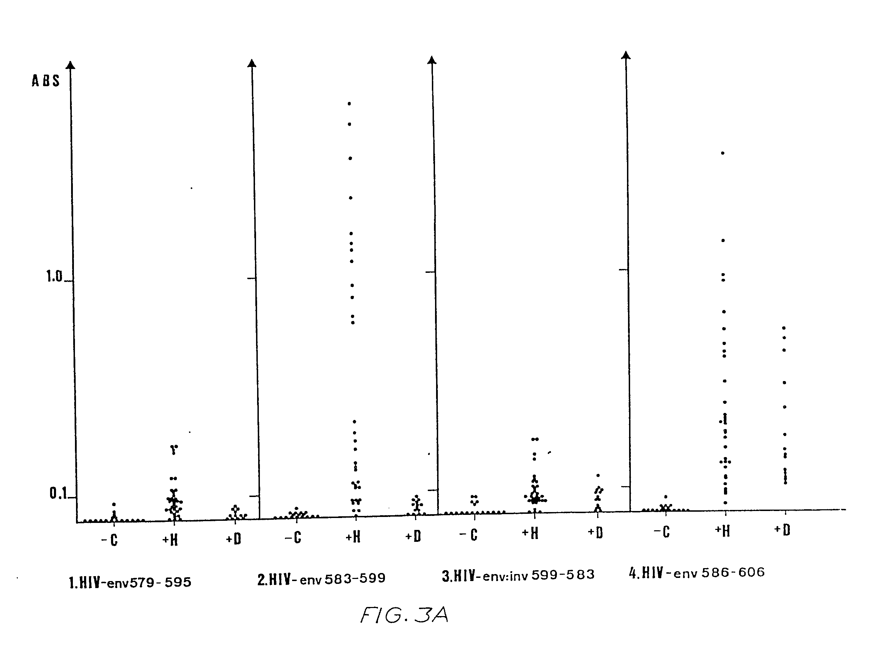

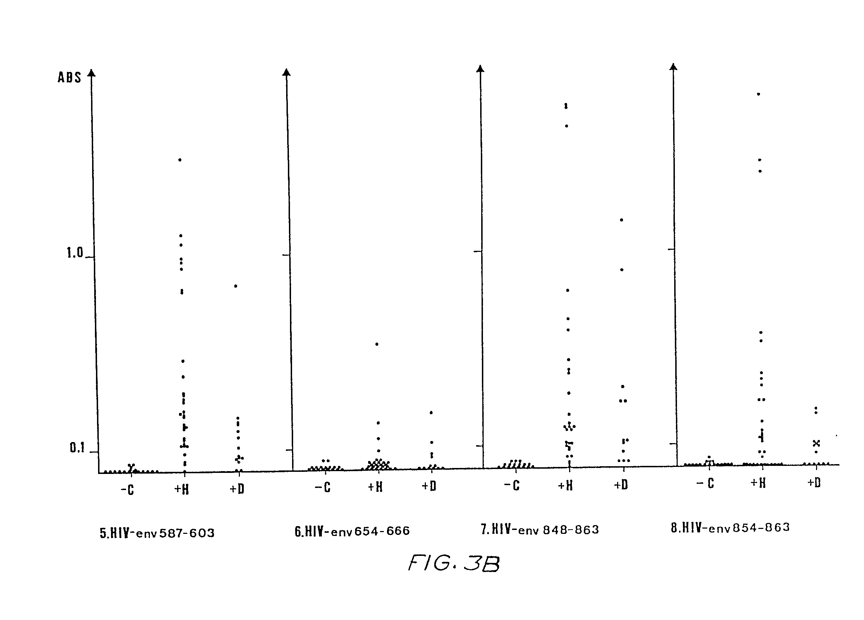

The absorbance values were consitently not normally distributed in the three serum categories; the logarithmic absorbances were inconsistently so. Therefore differences between healthy and diseased subjects were analysed as proportions above and below the cutoff value (with Fisher's exact test) and non-parametrically (with Wilcoxon's rank sum test). All original data and statistical test results are given i Figures 1, 2 and 3. Since the statistical tests were repeated eight times we corrected the p-value of high significance, 0.001, by dividing with 8 (Bonneferoni's correction, (28)). Thus the difference between healthy and diseased HIV-positives turned out to be highly significant for only one peptide: pHIVIS. 25 out of 32 healthy HIV-positives reacted with this peptide, whereas none of the 14 with illness did, the highest value in the latter group being 0.089 absorbance units. Wilcoxon's rank sum test for the difference between these two groups gave a p-value of $7.8 \times 10^{-6} \ll 0.001/8$ Fisher's exact test yielded $p = 1.3 \times 10^{-6} \ll 0.001/8$

Comparison with other measures of HIV seropositivity

We checked the possibility that high absorbance values for pHIVIS were a mere reflection of abundance of gp41-antibodies. Densitometry was performed on EIB-strips of a random sample of 8 sera positive to and 8 negative (of which 6 were ill) to HIV-env 583-599: The average absolute refelctance values (expressed in color yield units, (26) were 2.0 for both groups.

A random sample of eight sera reactive and six unreactive with pHIVIS was titrated from $4 \times 10^{-2}$ to $4 \times 10^{-6}$ in a commercial HIV antibody EIA. The average $^{10}$log of the titer was -2.4 for the first group and -2.0 for the second.

DISCUSSION

Theoretically, synthetic and recombinant peptides are excellent tools for mapping of epitopes and exploration of the individuality of the immune response (28-30). Recombinant and synthetic peptides corresponding to subsequences of gp41 have been used in solid phase immunoassays for detection of antibodies to HIV and to identify vaccine-relevant epitopes (18, 31-33). However, the prerequisites for definition of an epitope by use of short synthetic peptides under these circumstances are somewhat unclear. In their interaction with hydrophobic surfaces the conformationally unstable oligopeptides may be locked in configurations which favor or disfavor binding of natural antibodies. Thus, the conditions will select against antibodies not only to many discontinuous but also some of the continuous epitopes in the respective regions of gp41. With these reservations in mind we have studied the extent of IgG antibody reactivity to peptides from three regions of HIV gp41 which for several reasons are worthy of special attention. The putatively immunosuppressive sequence of HIV (pHIVIS) was the main target of our investigation. Antibodies binding to pHIVIS have great potential both immediately as indicators of disease progression and eventually as therapeutic agents. It was then very interesting to find that we could demonstrate such antibodies, that their presence was strongly associated with health, and that this association was essentially confined to a narrow region of gp41 corresponding to pHIVIS. Several controls were made to exlude trivial causes of this phenomenon.

First, by the use of synthetic oligopeptides prepared from different portions of gp41 as well as an other anti-gp41 parameter, quantification of the gp41 antibody band in EIB, we showed that a general decline of anti-gp41 activity was not a likely reason for our findings. There was however a small difference between pHIVIS-positives and -negatives in EIA titrations, and the absorbance values against most peptides tended to be somewhat lower in the diseased group. All this is in line with the reports that anti-gp41 antibody levels do not decline as much as anti-p24 antibody levels when progression to AIDS takes place (34).

Second, to control for nonspecific reactions all sera were tested in parallell against pHIVIS and its inverted counterpart. Reactions with minor byproducts of the synthesis could be detected in this way. We did not expect any specific reactions with this peptide. However, theoretically such reactions could arise from sequence specific antibodies with a very low dependence on antigen conformation. Interestingly, although inversion of the sequence lowered the rate and intensity of reactivity considerably, the weak reactions which were observed were still completely HIV-specific as no HIV negative controls reacted. 3 sera that reacted weakly with the inverted peptide were unreactive with pHIVIS, which could reflect cross-reactivity with the native protein in an infrequent conformation.

In an attempt to map the bounds of an epitope involving pHIVIS, its reactivity was compared with the respective reactivities of N- and C-terminally displaced peptides: HIV-env 579-595 yielded both low intensities and a low rate of reactivity. However, in only one case was the reactivity with HIV-env 579-585 above cutoff when it was not for pHIVIS, and the difference was only 0.045 absorbance units. The C-terminal displacement (587-603) increased the rate of reactivity. It was above cutoff in 14 sera that were unreactive with pHIVIS. Vice versa three cases were positive for HIV-env 583-599 (pHIVIS) but not for HIV-env 587-603. We therefore suggest that the C-terminal displacement causes the inclusion of one distinct epitope and the partial loss of another. It was when all 6 amino acids which are identical to the immunosuppressive MuLV sequence (LQ.R.LK.L) (14) were included that we got the highest differentiation between healthy and diseased individuals. This also included both hydrophilicity maxima in the region. Data are thus compatible with the existence of an epitope in the region of gp41 that is moderately similar to the immunosuppressive sequences in the transmembrane envelope glycoproteins of other retroviruses. Recent results suggest that also pHIVIS has immunosuppressive properties in vitro (G. Cianciolo, personal communication). Against this background

6

the statistically highly significant difference in pHIVIS reactivity between sera from healthy and diseased HIV-positive persons can be interpreted in one or several of the following ways: Antibodies to this part of gp41 have a protective effect; some HIV-infected persons may never raise an antibody response to this epitope and thereby lack protection from pHIVIS-induced immunosuppression.

Alternatively our failure to detect antibodies against this epitope in the diseased may have been due to formation of complexes between antibodies and a hypothetical circulating gp41-derived immunosuppressive factor. However, as mentioned, there is no great decline in anti-gp41 antibody levels during clinical progression (34). Our finding could thus be reconciled with either a selective decline within the whole set of gp41-antibodies or a genetic predisposition not to form antibodies to the epitope in question.

The longest peptide used here, HIV-env 586-606, showed the highest rate of reactivity with both sera from healthy and diseased HIV-positive subjects. This is in accordance with the previously reported results of Wang et al. A shorter peptide (HIV-env 587-603) within the same amino acid stretch gave a lower rate of reactivity, but discriminated somewhat between healthy and diseased patients. That points to an important contribution by the C-terminal Gly, Cys, Ser in the cross reaction with a major epitope, recognized regardless of state of health, of the native protein.

A 13-amino acid stretch (HIV-env 654-666) was recently predicted (19) to be an important antigenic determinant. This peptide was shorter than six of the other peptides, which may be part of the explanation why it gave fewest and weakest reactions. Nevertheless, if the EIA conditions, requiring hydrophobic interaction with plastic, were not unfavorable for testing it, it does not seem to correspond to a highly immunogenic epitope.

The shortest peptide (HIV-env 854-863) gave more frequent and stronger reactions than HIV-env 654-666, albeit not as many as the N-terminally extended HIV-env 848-863. Five of the six C-terminal amino acids in these peptides are identical to a putative receptor binding site of interleukin-2 (20). This is a relatively conserved region, but different isolates have the IL-2 similarity to a greater (SF2, HTLVIIIb) or smaller (NY5) degree. Many sera recognized one or more epitopes in this area. Whether the amino acids matching IL-2 are involved in the antibody binding site remains an open question and so does the pathogenetic potential of the sequence similarity. Anyhow we did not discern any difference between healthy and diseased subjects in their reaction patterns to the peptides from this region.

In conclusion, there was a considerable variation between individual sera in their reaction patterns to peptides derived from different parts of the gp41 sequence. Some of this variation may be genetically programmed (35) and may affect the progression to disease. Antibody reactivity to pHIVIS was strongly associated with health. Future studies ought to elucidate the role of pHIVIS in HIV pathogenesis. Such antibodies could be exploited therapeutically. The presence of anti-pHIVIS antibodies could be a useful prognostic test in HIV infected persons.

## Dissection of human antibody reactivities to a region of the HIV-1 transmembrane protein

Some peptides derived from gp41, the transmembrane protein of HIV-1 (human immunodeficiency type 1), can be used to detect antibodies in sera from nearly all persons who are HIV-1-seropositive by Western blot (40, 44, 47). One peptide, also derived from gp41, was found to react more often with antibodies from symptomless than from ill HIV-seropositive persons (as shown above) and is recently reported to inhibit allogeneic and CD3 stimulation of lymphocytes in vitro (37). The same peptide has six exact amino-acid matches with an in vitro-immunosuppressive peptide derived from the transmembrane protein of the murine leukemia virus (14). If the in vitro effect of the HIV peptide corresponds to a pathogenic mechanism, then antibodies reactive with the peptide might be therapeutically interesting. Recently, a point mutation in this region was found to confer escape from neutralization by a human serum (45). We derived six peptides from this region and compared them serologically with sixteen single-deletion peptides, and with ten peptides containing substituting threonine pairs. Threonine was chosen since a mutation to threonine (or serine) is found to be the change that, on average, natural selection allows most often (38). The results indicate the amino acids that are most important in antibody binding to this part of gp41. The different effects of changes in the amino-acid sequence on the antibody reactivity of different human sera illustrate the vagueness of the epitope concept in a polyclonal context: an epitope can only be exactly defined by a monoclonal antibody (36).

## Sera

We used sera from 21 symptom-free patients, confirmed by Western blot to have antibodies against HIV-1, and from 10 blood donors found by EIA (enzyme immuno-assay) to be HIV-seronegative. 17 of the HIV-positive sera were previously found to react with HIV-env 583-599 and were further tested against the substitution and deletion peptides.

## Peptide synthesis

Amino-acid sequences were derived from the nucleotide sequence of the clone HXB2 (21). Table 1 shows the sequences of the peptides. They were synthesized in the same way as described above or by Houghten's method of simultaneous multiple peptide synthesis (42). The HPLC showed a purity above 98% for all the peptides. The proportions of amino acids were checked by ion-exchange chromatography.

### Enzyme immunoassay, EIA

Microtiter plates (highly activated Titertek Immunoplates, Flow Laboratories, Aberdeen, Great Britain) were coated with peptides (10 μg/ml) or mock-coated with only phosphate-buffered saline. The assay was done in the same way as described above. For statistical tests of the results we used the Microstat package (Ecosoft, Indianapolis, IN) and for secondary structure predictions the Garnier algorithm (39) in the computer program "PC Gene" (Genofit, Geneva, Switzerland).

The surface occupancies of the peptides were compared by competition with $^{125}$I-labeled bovine serum albumin. At the concentrations used all peptides competed approximately to the same extent with the albumin.

### EIA results

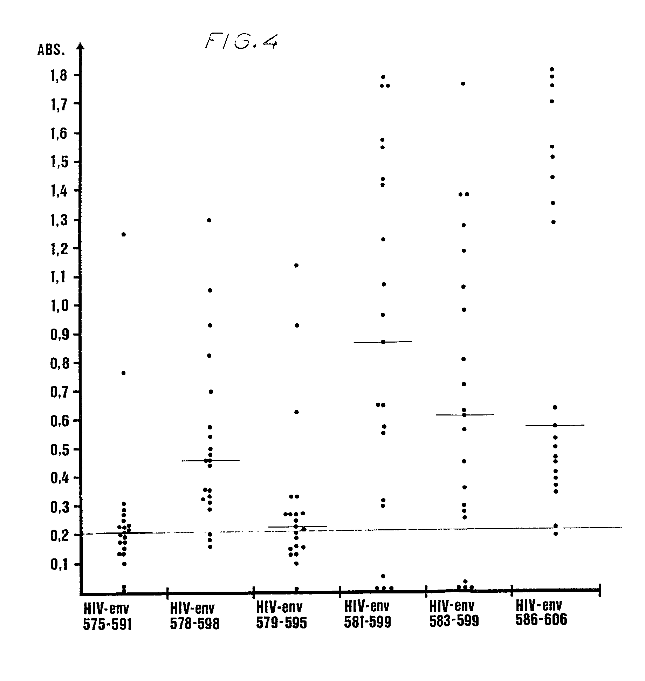

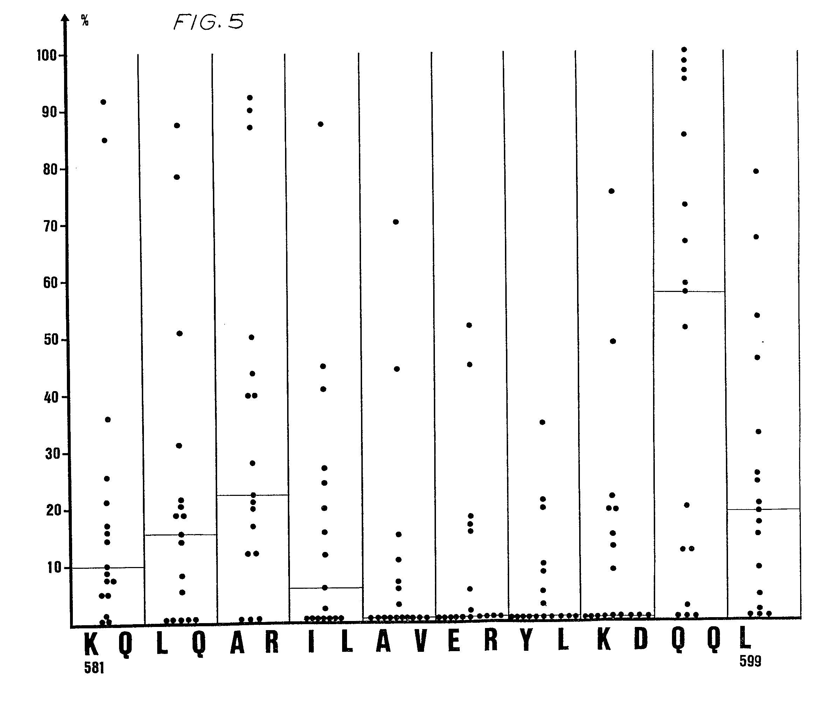

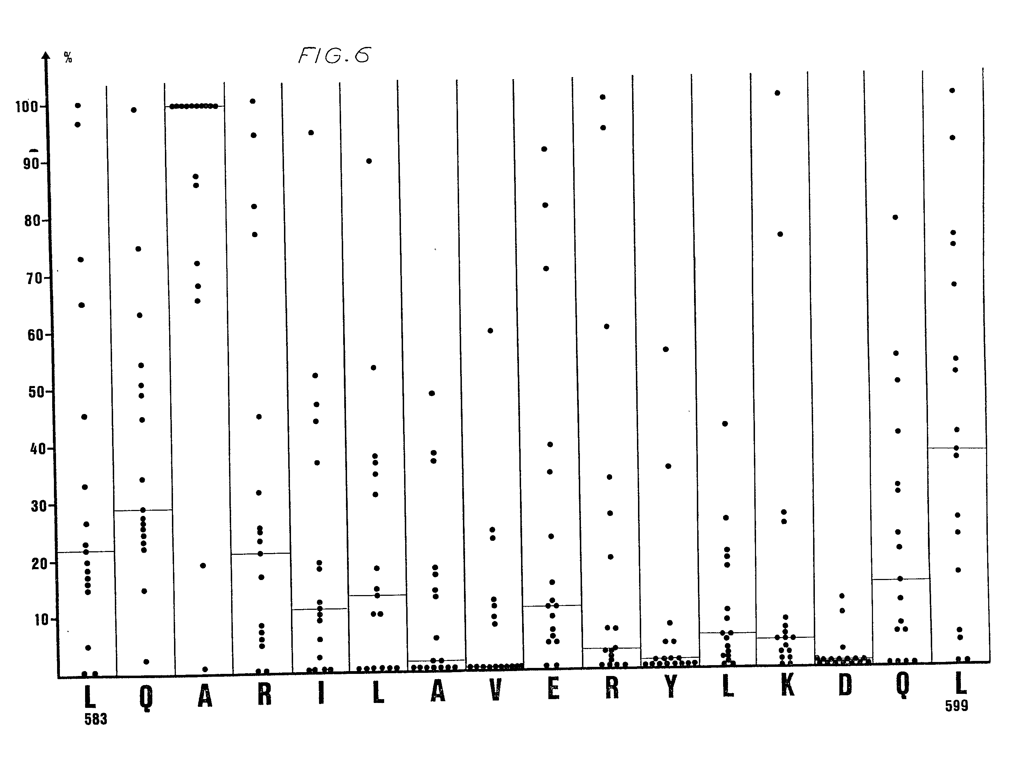

The absorbances of the mock-coated wells were substracted from those of the peptide-coated wells. Thus obtained absorbance differences were below 0.1 absorbance units for all the HIV-negative sera. Figure 4 gives the reactivities of the 21 HIV-positive sera with the peptides derived directly from the nucleotide sequence of HIV. Figures 5 and 6 show the results of the substitutions and deletions.

The reactivities of the sera with each peptide were ranked. The ranked reactivities were checked for correlations with each other. The reactivities of most of the unmodified peptides did not correlate with each other, thus indicating that these peptides do not simulate the same epitope. The exceptions were HIV-env 575-591 and 579-595 (r=0.85), 578-598 and 586-606 (r=0.71), 581-599 and 586-606 (r=0.71). We could, however, not dissect any distinct amino- or carboxy-terminally located determinants; the reactivities of the amino-terminally shifted peptides did not correlate stronger with reactivites of carboxy-terminally than with amino-terminally modified peptides, or vice versa. Highest correlations were found between reactivities of peptides with contiguous primary-structural changes (coefficients not shown); deletions of amino acids with side chains oriented next to each other in a helix yielded uncorrelated reactivites.

The evolutionary neutrality (38) of the threonine-pair substitutions did not correlate with the median relative reactivities: a high correlation would have been expected if all amino acids were equally important for the antigenicity of the peptide.

In accordance with a previous report (47) all of the HIV-positive sera reacted to some degree with HIV-env 586-606. HIV-env 581-599 gave higher reactivities that HIV-env 583-599 (Figure 4). (We have, when screening other sera, found some that react strongly with HIV-env 581-599 but not at all with HIV-env 583-599 (unpublished)). Most of the sera were also positive for the amino-terminally shifted 21-mer, HIV-env 578-598 (Figure 4). The difference in reactivity between HIV-env 578-598 and HIV-env 581-599 was above 0.5 absorbance units in 10 cases: in 9 of these it was higher with HIV-env 581-599. This shows the importance of the leucine in position 599, the only amino acid that is in HIV-env 581-599 but not in HIV-env 578-598. The substitution of threonine for leucine in this position lowered the reactivity of the peptide (Figure 5), with a median reduction of 80%. The deletion of this leucine gave a medium decrease in the reactivity with HIV-env 583-599 of 61% (Figure 6).

Fig 5 shows that the substitutions in the middle amino acids in the peptide HIV-env 581-599 had the most drastic serological effects; exchanges in the sequence Ala-Val-Glu-Arg-Tyr-leu-Lys-Asp 589-596 (see Figure 4) reduced the reactivities with 100% in over half of the cases. With deletions in this sub-sequence the median reductions were above 88%; deleting the aspartic acid in position 596 gave the greatest reduction in reactivity of all the changes studied. This stretch is predicted to form an alpha helix in the whole protein as well as in the five unmodified peptides encompassing it (Table 1). According to the predictions for the modified peptides, reductions in antigenicity do not seem to be solely due to changes in secondary structure; changes in the stretch 589-596 that were not predicted to break the alpha helix reduced the antigenicity more than changes outside it (see Table 1).

Conjugating iodine to the tyrosine in position 593 does not abolish antibody binding (unpublished data). Still, deleting this tyrosine abrogated the reactivity of most sera, and so did the threonine-threonine substitution for tyrosine and leucine. All this could be explained by a conformational effect of this tyrosine without its interacting directly with the antibody. According to the prediction, however, the deletion of tyrosine stabilizes the alpha helix. Moreover, if the peptide HIV-env 583-599 is helical, then the the side chains of alanine in position 589, valine, tyrosine, and aspartic acid would be within the same third of the helix circumference. Deletions of these amino acids also have the most drastic effects on antibody reactivity. This possibly indicates the side of the helix that interacts most intimately with the antibodies.

The threonines in positions 581-582 reduced the reactivity with over 60% for 15 of the 17 sera, i e less than the reactivity with HIV-env 583-599. This could be due to a conformational disturbance by these two threonines, although their predicted effect on secondary structure is but slight (see Table 1).

Substituting threonine-threonine for glutamine-glutamine in positions 597-598 affected antibody binding less than all other substitutions, even less than the single threonine at the C terminus: this could mean that the two glutamine side-chains are directed away from the antibody-binding surface. Deleting one of these glutamines affected the reactivity more than substituting a pair of threonines for both of them.

The change preservering the reactivities most was the deletion of the alanine in position 585. Still, this deletion illustrated the individuality of the antibody response: it reduced the reactivity of one serum to 1%, of another to 19%; whereas it did not affect the reactivity of ten sera. In contrast, deletion of the other alanine, in position 589, gave a median relative reactivity of only 2%: interestingly, the substitution of threonine for alanine in the latter position can impede neutralization by a polyclonal serum (45). Such a substitution lowers the probability of an alfa-helix in the region and makes a beta sheet and a beta turn more probable, as predicted by the algorithm of Garnier et al. (39, 43). Apparently, a change in this position affects both the envelope function, as shown by the neutralization experiments (45), and the antigenicity of a synthetic peptide, as indicated by our serological data. Although antibodies to a peptide derived only somewhat more carboxyterminally from the sequence of gp41 are neutralizing (46), the neutralization that the change in position 589 abrogates is not necessarily due to the binding of antibodies to this region. It also remains to be seen whether the peptide-reactive antibodies reported here have any effect on the pathogenicity or the spread of HIV-1.

Abbreviations:
HIV = human immunodeficiency virus; LAS = lymph adenopathy syndrome,considered as synonymous to PGL = Peripheral glandular lymph adenophaty; ARC = AIDS-related complex of syndromes; AIDS = acquired immunodeficiency syndrome.

## TABLE 1

### Amino acid sequences

The amino acids predicted by the algorithm of Garnier et al. (39) to be part of extended secondary structures have been underlined, those of beta turns have been doubly underlined; the rest are predicted to be part of alpha helices.

Amino acid position[x]        sequence#

Region studied (part of pre-
diction for whole protein):

| | |
|---|---|
| HIV-env 575-606 | LTVWGIKQLQARILAVERYLKDQQLLGIWGCS |

Synthetic peptides:

| | |
|---|---|
| HIV-env 575-591 | LTVWGIKQLQARILAVE |
| HIV-env 578-598 | WGIKQLQARILAVERYLKDQQ |
| HIV-env 579-595 | GIKQLQARILAVERYLK |
| HIV-env 581-599 | KQLQARILAVERYLKDQQL |
| HIV-env 583-599 | LQARILAVERYLKDQQL |
| HIV-env 586-606 | RILAVERYLKDQQLLGIWGCS |

substitutions in        median
HIV-env 581-599:       relative
             reactivity:

| | | | | | |
|---|---|---|---|---|---|
| TT[x] | in position 581-582 | | TTLQARILAVERYLKDQQL | 10% |
| " | " | " | 583-584 | KQTTARILAVERYLKDQQL | 15% |
| " | " | " | 585-586 | KQLQTTILAVERYLKDQQL | 23% |
| " | " | " | 587-588 | KQLQARTTAVERYLKDQQL | 6% |
| " | " | " | 589-590 | KQLQARILTTERYLKDQQL | 0% |
| " | " | " | 591-592 | KQLQARILAVTTYLKDQQL | 0% |
| " | " | " | 593-594 | KQLQARILAVERTTKDQQL | 0% |
| " | " | " | 595-596 | KQLQARILAVERYLTTQQL | 0% |
| " | " | " | 597-598 | KQLQARILAVERYLKDTTL | 57% |
| T | " | " | 599 | KQLQARILAVERYLKDQQT | 19% |

10

Deletions from HIV-env 583-599:

Deletion in

| position | | | |
|---|---|---|---|
| 583 | -QARILAVERYLKDQQL | 22% | |
| 584 | L-ARILAVERYLKDQQL | 28% | |
| 585 | LQ-RILAVERYLKDQQL | 100% | |
| 586 | LQA-ILAVERYLKDQQL | 21% | |
| 587 | LQAR-LAVERYLKDQQL | 11% | |
| 588 | LQARI-AVERYLKDQQL | 13% | |
| 589 | LQARIL-VERYLKDQQL | 2% | |
| 590 | LQARILA-ERYLKDQQL | 0.5% | |
| 591 | LQARILAV-RYLKDQQL | 11% | |
| 592 | LQARILAVE-YLKDQQL | 4% | |
| 593 | LQARILAVER-LKDQQL | 2% | |
| 594 | LQARILAVERY-KDQQL | 6% | |
| 595 | LQARILAVERYL-DQQL | 5% | |
| 596 | LQARILAVERYLK-QQL | 1% | |
| 597 or 598 | LQARILAVERYLKD-QL | 15% | |
| 599 | LQARILAVERYLKDQQ- | 38% | |

☆ The amino acids derived from the _env_ gene of the HIV isolate were numbered according to Ratner et al. (21).

# A, alanine; C, cysteine; D, aspartic acid; E, glutamic acid; G, glycine; H, histidine; I, isoleucine; K, lysine; L, leucine; N, asparagine; P, proline; Q, glutamine; R, arginine; S, serine; T, threonine; V, valine; W, tryptophan; Y, tyrosine.

REFERENCES
1. Lifson J.D., Reyes G.R., McGrath M.S., Stein B.S. & Engleman E.G. (1986) Science 232, 1123-1127.
2. Sodroski J., Chun Goh W., Rosen C., Campbell K. & Haseltine W.A. (1986) Nature (London) 322, 470-474.
3. Lifson J.D., Feinberg M.B., Reyes G.R., Rabin L., Banapour B., Chakrabarti S., Moss B., Wong-Staal F., Steimer K.S. & Engleman E.G. (1986) Nature (London) 323, 725-728.
4. Fisher A.G., Ratner L., Mitsuya H., Marselle M., Harper M.E., Broder S., Gallo R.C. & Wong-Staal F. (1986) Science 233, 655-659.
5. Pahwa S., Pahwa R., Saxinger C., Gallo R.C. & Good R.A. (1985) Proc. Natl. Acad. Sci. USA 82, 8198-8202.
6. Pahwa S., Pahwa R., Good R.A., Gallo R.C. & Saxinger C. (1986) Proc. Natl. Acad. Sci. USA 83, 9124-9128.
7. Lane H.C., Masur H., Lynn L.C., Whalen G., Rook A.H. & Fauci A.S. (1983) N. Engl. J. Med. 309, 453-458.
8. Schnittmann S.M., Lane H.C., Higgins S.E., Folks T. & Fauci A.S. (1986) Science 233, 1084-1086.
9. Laurence J., Gottlieb H.B. & Kunkel H.G. (1983) J. Clin. Invest. 72, 2072-2081.

10. Laurence J. & Mayer L. (1984) Science 225, 66-69.

11. Fowler A.K., Twardzik D.R., Reed C.D., Weislow O.S. & Hellman A. (1977) Cancer Research 37, 4529-4531.

12. Mathes L.E., Olsen R.G., Hedebrand L.C., Hoover E.A. & Schaller J.P. (1978) Nature (London) 274, 687-689.

13. Mathes L.E., Olsen R.G., Hedebrand L.C., Hoover E.A., Schaller J.P., Adams P.W. & Nichols W.S. (1979) Cancer Research 39, 950-955.

14. Cianciolo G.J., Copeland T.D., Oroszlan S. & Snyderman R. (1985) Science 230, 453-455.

15. Cianciolo G.J., Hunter J., Silva J., Haskill J.S. & Snyderman R. (1981) J. Clin. Invest. 68, 831-844

16. Mitani M., Cianciolo G.J., Snyderman R., Yasuda M., Good R.A. & Day N.K. (1987) Proc. Natl. Acad. Sci. USA 84, 237-240.

17. Harris D.T., Cianciolo G.J., Snyderman R., Argov S. & Koren H.S. (1987) J. Immunol. 138, 889-894.

18. Wang J.J.G., Steel S., Wisniewolski S. & Wang C.Y. (1986) Proc. Natl. Acad. Sci. USA 83, 6159-6163.

19. Robson B., Fishleigh R.V. & Morrison C.A. (1987) Nature 325, 395.

20. Reiher III W.E., Blalock J.E. & Brunck T.K. (1986) Proc. Natl. Acad. Sci. USA 83, 9188-9192.

21. Ratnher L., Haseltine W., Patarca R., Livak K.J., Starcich B., Josephs S.F., Doran E.R., Rafalski J.A., Whitehorn E.A., Baumeister K., Ivanoff L., Petteway Jr S.R., Pearson M.L., Lautenberger J.A., Papas T.S., Ghrayeb J., Chang N.T., Gallo R.C. & Wong-Staal F. (1985) Nature 313, 277-284.

22. Merrifield R.B. (1963) J. Am. Chem. Soc. 85, 2149-2154

23. Wang S.S. (1973) J. Am. Chem. Soc. 95, 1328-1333

24. Anonymous (1986) NMWR 35, 334-339

25. Blomberg J., Nilsson I. & Andersson M. (1983) J. Clin. Microbiol. 17, 1081-1091.

26. Blomberg J. & Klasse P.J. (1988) J. Clin. Microbiol. 26, 111-115.

27. Hopp T.H. (1986) J. Immunol. Meth. 88, 1-18.

28. Godfrey K. (1985) N. Engl. J. Med. 313, 1450-1456.

29. Novotny J. & Handschumacher M. & Bruccoleri R.E. (1986) Immunol. Today 8, 26-31.

30. Berzofsky J.A. (1985) Science 239, 932-940.

31. Kennedy R.C., Henkel R.D., Pauletti D., Allan J.S., Lee T.H., Essex M. & Dreesman G.R. (1986) Science 231, 1556-1559.

32. Certa U., Bannwarth W., Stuber D., Gentz R., Lanzer M., Le Grice S., Guillot F., Wendler I., Hunsmann G., Bujard H. & Mous J. (1986) EMBO J. 5, 3051-3056.

33. Crowl R., Ganguly K., Gordon M., Conroy R., Schaber M., Kramer R., Shaw G., Wong-Staal F. & Reddy E.P. (1985) Cell 4, 979-986.

34. Lange J.M.A., Coutinho R.A., Krone W.J.A., Verdonck L.F., Danner S.A., van der Noordaa J. & Gcudsmit J. (1986) Brit Med J 292, 228-230.

35. Unanue E.R. & Allen P.M. (1987) Science 236, 551-557.

36. Barlow D.J., Edwards M.S., and Thornton J.M., (1986). Nature 322: 747-748.

37. Cianciolo G.J, Bogerd H., Snyderman R., (1988). Immunol. Lett. 19: 7-13.

38. Dayhoff M.O., Schwartz R.M., and Orcutt B.C., (1988). A model of evolutionary change in proteins, p. 345-352. In Dayhoff M.O. (ed.), Atlas of protein sequence and structure vol. 5, suppl 3, National Bio-medical Research Foundation, Washington, D.C.

39. Garnier J., Osguthorpe D.J., and Robson B., (1978). J. Mol. Biol. 120: 97-120.

40. Gnann J. W., Schwimmbeck Jr, P.L., Nelson J.A., Truax A.B., Oldstone M.B.A, (1987). J. Inf. Dis. 156: 261-267.

41. Gnann J.W., Nelson J.A., and Oldstone M.B.A, (1987). J. Virol 61: 2639-2641.

42. Houghten R.A. (1985). Proc. Natl. Acad. Sci. 82:5132-5135.

43. Kabsch W., Sander C., (1983). FEBS Lett 155: 179-182.

44. Närvänen A., Korkolainen M., Kontio S., Suni J., Turtianen S., Partanen P., Soos J., Vaheri A., and Huhtala M.L., (1988). AIDS 2: 119-123.

45. Reitz Jr M.S., Wilson C., Naugle C., Gallo R.C., Robert-Guroff M., (1988). Cell 54: 57-63.

46. Schrier R.D., Gnann Jr J.W., Langlois A.J., Shriver K., Nelson J.A., Oldstone M.B.A, (1988), J. Virol 62: 2531 2536.

47. Wang J.J.G., Steel S., Wiesniewolski R., and Wang C.Y., (1986). Proc. Natl. Acad. Sci.. 83: 6159-6163.

LEGENDS FOR FIGURES

Figure 1

Frequency of IgG antibody reactivity with eight different peptides derived from the HIV gp41 sequence. The numbers at the top of the Figure correspond to the 8 peptides: 1 = HIV-env 579-585, 2 = HIV-env 583-599 (pHIVIS), 3 = "HIV-env 599-583", 4 = HIV-env 586-606, 5 = HIV-env 587-603, 6 = HIV-env 654-666, 7 = HIV-env 848-863 and 8 = HIV-env 854-863. In the left margin each serum is symbolized by a tick mark; the sera are grouped according to clinical status of the subjects, as indicated. Absorbance values above the cutoff (see text) are represented as striped rectangles in the healthy group and as chequered ones in the group with disease. The difference between the two groups in proportions above and below cutoff was tested by means of Fisher's exact test, the level of high statistical signficance being adjusted to $p < 0.001/8 = 1.25 \times 10^{-4}$. 1:HIV-env 579-585 10/32 versus 0/14, $p = 0.016$. 2: HIV-env 583-599 (pHIVIS) 24/32 versus 0/14, $p = 1.26 \times 10^{-6}$. 3:

"HIV-env 599-583", 10/32 versus 2/14, p=0.20. 4: HIV-env 586-606, 29/32 versus 14/14, p=0.33. 5: HIV-env 587-603, 28/32 versus 7/14, p=0.010. 6: HIV-env 654-666, 3/28 versus 2/12, p=0.48. 7: HIV-env 848-863, 22/28 versus 8/12, p=0.34. 8: HIV-env 854-863, 17/32 versus 5/14, p=0.22. * =not done.

Figure 2

Distribution of antibody reactivity in helathy and diseased HIV seropositive persons with the HIV gp41 peptides in relation to their sequence position and hydrophilicity. the Hopp-Woods' indices (26), integrated as a moving average over six amino acids, are plotted on the ordinates. On the abscissas the polypeptide sequences are depicted for each of the three regions that the peptides were derived from. Underneath the synthesized peptides are represented in their respective positions along the sequence. In the diagrams the peptides are also symbolized as arrows from N- to C-terminal and numbered as in Fig. 1. Below each arrow are two bars, the upper one corresponding to healthy, the lower one to diseased cases. Each bar is filled with black proportionally to the numbers of sera that gave absorbance values above cutoff in the respective categories.

Part A: HIV-env 576-609. Part B: HIV-env 651-670. Part C: HIV-env 845-863.

Figure 3

Quantitative distribution of reactivities to the eight peptides. The absolute absorbance values after background subtraction are represented on the ordinate for each peptide. -D=negative control sera, +H=HIV-positive sera from healthy individuals and +D=HIV-positive sera from patients with HIV-related disease. Wilcoxon's rank sum test was performed to compare the absorbances of categories #H and #D for each peptide, $p < 0.001/8 = 1.25 \times 10^{-4}$ regarded as statistically highly significant.

Part A: 1; HIV-env 579-595, $p < 1.6 \times 10^{-4}$. 2; HIV-env 583-599 (pHIVIS), $p < 7.8 \times 10^{-6}$. 3; "HIV-env:inv. 599-573", $p < 0.093$. 4; HIV-env 586-606, $p < 0.30$.

Part B: 5; HIV-env 587-603, $p < 2.9 \times 10^{-3}$. 6; HIV-env 654-666, $p < 0.23$. 7; HIV-env 848-863, $p < 0.28$. 8; HIV-env 854-863, $p < 0.24$.

Figure 4

The EIA reactivities (background subtracted) for the 21 HIV-poisitive sera with the five peptides derived from the nucleotide sequence of the HIV1 env gene. The reactivities are expressed as optical density at 450 nm. Horizontal lines indicate the median values.

Figure 5

The EIA reactivities with the threonine-substituted peptides for the 17 sera positive for HIV-env 581-599. Each peptide is represented on the base line by the the symbols (see Table 1) of the amino acids for which threonine was substituted. Thus the whole sequence of HIV-env 581-599 can be read on the base line. The reactivities with the modified peptides are expressed as percentages of the respective reactivities with HIV-env 581-599. Horizontal lines indicate the median values.

Figure 6

The EIA reactivities with the deletion peptides for the 17 sera positive for HIV-env 583-599. Each peptide is represented by the symbol of its deleted amino acid on the base line. Notice that only one Q (for glutamine) is given in the second from last position: since there is a pair of glutamines in positions 597-598, deleting either of these results in the same peptide. The reactivites with the modified peptides are expressed as percentages of the reactivities with HIV-env 583-599. Horizontal lines indicate the median values.

**Claims**

1. A peptide having the amino acid sequence
H-Leu-Gln-X-Arg-Ile-Leu-Ala-Val-Glu-Arg-Tyr-Leu-Lys-Asp-Y-Z-Leu-W

wherein
X represents a peptide bond or an amino acid residue selected from Ala, Thr, Val, Ser and Asn;
Y represents a peptide bond or an amino acid residue selected from Gln, Gly, Ala and Thr;
Z represents a peptide bond or an amino acid residue selected from Gln, Gly, Ala and Thr; and
W represents -OH or -NH$_2$.

2. A peptide for diagnostic use in the determination of possible development in an HIV infected patient of a disease caused by HIV, such as LAS, ARC or AIDS, **characterised** in that it is chosen from the group consisting of peptides having the amino acid sequence

H-Leu-Gln-X-Arg-Ile-Leu-Ala-Val-Glu-Arg-Tyr-Leu-Lys-Asp-Y-Z-Leu-W

wherein
X represents a peptide bond or an amino acid residue selected from Ala, Thr, Val, Ser and Asn;
Y represents a peptide bond or an amino acid residue selected from Gln, Gly, Ala and Thr;
Z represents a peptide bond or an amino acid residue selected from Gln, Gly, Ala and Thr; and
W represents -OH or -NH₂.

3. An antibody reacting with at least one peptide chosen from the group consisting of peptides having the amino acid sequence

H-Leu-Gln-X-Arg-Ile-Leu-Ala-Val-Glu-Arg-Tyr-Leu-Lys-Asp-Y-Z-Leu-W

wherein
X represents a peptide bond or an amino acid residue selected from Ala, Thr, Val, Ser and Asn;
Y represents a peptide bond or an amino acid residue selected from Gln, Gly, Ala and Thr;
Z represents a peptide bond or an amino acid residue selected from Gln, Gly, Ala and Thr; and
W represents -OH or -NH₂.

4. An antibody according to claim 3, wherein said antibody is a monoclonal antibody.

5. An antibody according to claim 3 or 4, wherein said antibody is coupled to a carrier.

6. A method of isolating antibodies reacting with at least one peptide chosen from the group consisting of peptides having the amino acid sequence

H-Leu-Gln-X-Arg-Ile-Leu-Ala-Val-Glu-Arg-Tyr-Leu-Lys-Asp-Y-Z-Leu-W

wherein
X represents a peptide bond or an amino acid residue selected from Ala, Thr, Val, Ser and Asn;
Y represents a peptide bond or an amino acid residue selected from Gln, Gly, Ala and Thr;
Z represents a peptide bond or an amino acid residue selected from Gln, Gly, Ala and Thr; and
W represents -OH or -NH₂;
from blood samples of HIV infected patients, **characterised** in that said blood samples are subjected to affinity chromatography.

7. A method of determining possible development in an HIV infected patient of a disease caused by HIV, such as LAS, ARC, or AIDS, with the aid of a sample of body fluid from said patient,
**characterised** in that said sample is subjected to an immunoassay using as diagnostic antigen at least one peptide chosen from peptides having the amino acid sequence

H-Leu-Gln-X-Arg-Ile-Leu-Ala-Val-Glu-Arg-Tyr-Leu-Lys-Asp-Y-Z-Leu-W

wherein
X represents a peptide bond or an amino acid residue selected from Ala, Thr, Val, Ser and Asn;
Y represents a peptide bond or an amino acid residue selected from Gln, Gly, Ala and Thr;
Z represents a peptide bond or an amino acid residue selected from Gln, Gly, Ala and Thr; and
W represents -OH or -NH₂;
and in case said sample of body fluid contains antibodies which bind to said diagnostic antigen, the patient is not likely to have a disease caused by HIV or develop one within a few years' time.

8. A method according to claim 7, wherein the immunoassay is enzyme linked immunosorbent assay (ELISA).

9. A method of detecting immunosuppressive protein produced by HIV in a sample of body fluid from an HIV infected patient, **characterised** in that said sample of body fluid is subjected to a competitive inhibition test using as a test peptide a radioactively, enzymatically or otherwise labelled peptide chosen from the group of peptides having the amino acid sequence

H-Leu-Gln-X-Arg-Ile-Leu-Ala-Val-Glu-Arg-Tyr-Leu-Lys-Asp-Y-Z-Leu-W

wherein
X represents a peptide bond or an amino acid residue selected from Ala, Thr, Val, Ser and Asn;
Y represents a peptide bond or an amino acid residue selected from Gln, Gly, Ala and Thr;
Z represents a peptide bond or an amino acid residue selected from Gln, Gly, Ala and Thr; and
W represents -OH or -NH₂;
and in case the binding of said test peptide to antibodies directed against at least one peptide of the above group of peptides is inhibited, said sample of body fluid contains immunosuppressive protein produce by HIV.

10. A medicament for prophylactic or therapeutic use in the prevention or treatment of a disease caused by HIV,
**characterised** in that the active principle of said medicament comprises antibodies reacting with at least one peptide chosen from the group consisting of peptides having the amino acid sequence

H-Leu-Gln-X-Arg-Ile-Leu-Ala-Val-Glu-Arg-Tyr-Leu-Lys-Asp-Y-Z-Leu-W

wherein
X represents a peptide bond or an amino acid residue selected from Ala, Thr, Val, Ser and Asn;
Y represents a peptide bond or an amino acid residue selected from Gln, Gly, Ala and Thr;
Z represents a peptide bond or an amino acid residue selected from Gln, Gly, Ala and Thr; and
W represents -OH or -NH$_2$.

11. A medicament according to claim 9, wherein said antibodies are polyclonal antibodies derived from blood samples of HIV infected human patients or apes.

12. A medicament according to claim 9, wherein said antibodies are monoclonal antibodies.

13. A vaccine composition, **characterised** in that it comprises as an immunizing component at least one peptide chosen from the group consisting of peptides having the amino acid sequence

H-Leu-Gln-X-Arg-Ile-Leu-Ala-Val-Glu-Arg-Tyr-Leu-Lys-Asp-Y-Z-Leu-W

wherein
X represents a peptide bond or an amino acid residue selected from Ala, Thr, Val, Ser and Asn;
Y represents a peptide bond or an amino acid residue selected from Gln, Gly, Ala and Thr;
Z represents a peptide bond or an amino acid residue selected from Gln, Gly, Ala and Thr; and
W represents -OH or -NH$_2$;
together with a nontoxic pharmaceutically acceptable carrier and/or diluent.

14. A vaccine composition according to claim 13, wherein said immunizing component is present in an amount effective to protect a subject from a disease caused by HIV.

15. A vaccine composition according to claim 13, wherein an adjuvant is included in an amount, which together with an amount of said immunizing component, is effective to protect a subject from a disease caused by HIV.

FIG.1

HOPP-WOODS' INDEX                    *FIG.2A*

HIV-env:576   580        590        600        610

TVWGIKQLQARILAVERYLKDQQLLGIWGCSGKL

1.HIV-env579-595 GIKQLQARILAVERYLK

2.HIV-env583-599    LQARILAVERYLKDQQL

3.HIV-env inv.599-583   LQQDKLYREVALIRAQL

4.HIV-env586-606        RILAVERYLKDQQLLGIWGCS

5.HIV-env587-603          ILAVERYLKDQQLLGIW

HOPP-WOODS' INDEX          FIG. 2B

6. HIV-env:654-666   E E S Q N Q Q E K N E Q E

**HOPP-WOODS' INDEX**          *FIG.2C*

7.HIV-env: 848-863

8.HIV-env: 854-863

FIG. 3A

5.HIV-env 587-603    6.HIV-env 654-666    7.HIV-env 848-863    8.HIV-env 854-863

FIG. 3B

EP 0 323 424 A2

FIG.4

FIG. 5

*FIG. 6*

% 100 90 80 70 60 50 40 30 20 10

L 583  Q  A  R  I  L  A  V  E  R  Y  L  K  D  Q  L 599